# EUROPEAN PATENT SPECIFICATION

(11) **EP 3 326 638 B1**
(45) Date of publication and mention of the grant of the patent: **15.01.2025**
(21) Application number: 16828006.3
(22) Date of filing: 15.07.2016
(51) Int. Cl.: A61P 1/04, A61P 17/00, A61P 19/10, A61K 36/738, A23K 10/30, A23L 33/105

(54) **COMPOSITIONS FOR USE IN PREVENTING OR TREATING IL-6-MEDIATED DISEASES COMPRISING ROSA RUGOSA**
ZUSAMMENSETZUNGEN ZUR VERWENDUNG ZUR VORBEUGUNG ODER BEHANDLUNG VON IL-6-VERMITTELTEN ERKRANKUNGEN MIT ROSA RUGOSA
COMPOSITIONS POUR L'UTILISATION POUR LA PRÉVENTION OU LE TRAITEMENT DE MALADIES MÉDIÉES PAR IL-6 COMPRENANT ROSA RUGOSA

(30) Priority: 17.07.2015 KR 20150101690
(43) Date of publication of application: 30.05.2018
(73) Proprietor: Korea Research Institute of Bioscience and Biotechnology, Daejeon 34141 (KR)
(72) Inventor: RHO, Mun Chual, Daejeon 34141 (KR); LEE, Seung Woong, Daejeon 34141 (KR); LEE, Woo Song, Daejeon 34141 (KR); KIM, Sang Hyun, Daegu 41944 (KR); LEE, So Young, Daejeon 34141 (KR); LEE, Seung Jae, Daejeon 34141 (KR); JUNG, Kyung Sook, Daejeon 34141 (KR)
(74) Representative: Gassner, Birgitta
(86) International application number: PCT/KR2016/007723
(87) International publication number: WO 2017/014502

(56) References cited:
- JP-A- 2006 316 026
- KR-A- 20130 090 935
- KR-A- 20140 045 893
- KR-A- 20140 081 325
- DATABASE WPI Week 201379, Derwent World Patents Index; AN 2013-M74278, XP002787008
- YOO, HYE SOO ET AL.: "The Study on Pharmacological Activation as Cosmetic Material of Rosa Rugosa Thunb. Flowers Extract", KOREAN JOURNAL OF ACUPUNCTURE, vol. 31, no. 4, 2014, pages 188 - 194, XP053033249
- KANG, SE CHAN ET AL.: "Effects of Fructus and Semen from Rosa Rugosa on Osteoimmune Cells", KOREAN JOURNAL OF PLANT RESOURCES, vol. 23, no. 2, April 2010 (2010-04-01), pages 157 - 164, XP053017527

## Description

### Technical Field

The present invention relates to a pharmaceutical composition for use in preventing or treating an IL-6-mediated disease, comprising a *Rosa rugosa Thunberg* flower extract as an active ingredient. Additionally, the present invention relates to a food composition, feed composition, and drinking water additive for use in preventing or improving an IL-6-mediated disease, comprising a *Rosa rugosa Thunberg* flower extract as an active ingredient.

### Background Art

Interleukin-6 (IL-6) is a cytokine also referred to as B cell stimulatory factor (BSF2) or interferon-2 (INF-2), and was discovered as a differentiation factor involved in activation of B lymphocytes (Nature 1986, 324, 73-76). IL-6 delivers its biological activities mediated by two different kinds of proteins on a cell membrane. One of the proteins serves as an IL-6 receptor, which is an IL-6-bound transmembrane protein having a molecular weight of about 80 kDa, whereas the other protein is a membrane protein gp130 with a molecular weight of about 130 kDa, which is involved in non-ligand binding signal transduction. IL-6 and IL-6 receptor together form an IL-6/IL-6 receptor complex, which then binds to gp130 (J. Exp. Med 1987, 166, 967). Upon binding between ligands and receptors, Janus Kinase 2 (JAK2) is activated by transphosphorylation in the cell. The activated JAK2 phosphorylates various tyrosine residues in cytoplasmic domains of the receptors, causing them to serve as docking sites for the intracellular proteins such as SH2 or signal transducers and activators of transcription 3 (STAT3) having phosphotyrosine binding motifs. The STAT3 bound to the cytoplasmic domain of a given receptor is released from the receptor after being phosphorylated by JAK2. The thus-activated STAT3 s bind to each other in the cytoplasm to form homodimers or heterodimers, enter the nucleus, and bind to the recognition sequence of a target gene, thereby increasing transcription (Nat Rev Mol Cell Biol 2002, 3, 651-62, Science 1997, 277, 1630-1635).

Signal transducer and activator of transcription 3 (STAT3) is known to play a role as an important transcription factor, in addition to NF-κB, in autoimmune diseases including rheumatoid arthritis or osteoporosis. STAT3 is particularly known to be activated by cytokine IL-6, while epidermal growth factor (EGF) is also known to be activated by IL-6. There have recently been many studies on the association between osteoporosis and IL-6; in particular, the fact that the STAT3 activation induced by IL-6 plays an important role in osteoclast differentiation and osteogenesis has been proven through animal models (Bone 2006, 39, 505-512), and in the case of STAT3-deficient mice, decreases in bone density and volume and an increase in the number of osteoclasts negatively influencing osteoporosis have been observed (BBRC 2005, 328, 800-807). In contrast, there have been reports that inhibition of bone resorption was observed in OVX mouse (an animal model for investigation of postmenopausal osteoporosis) treated with madindoline A, a compound which inhibits the IL-6-induced STAT phosphorylation (PNAS, 2002, 99(23), 14728-14733), an increase in the differentiation of osteoclasts from myeloblast cells by IL-6 was observed in estrogen-deficient osteoporosis models (Science, 1992, 257, 88-91), and no changes such as bone loss, an increase in the number of osteoclast precursors, *etc.* due to the estrogen deficiency were observed in IL-6-/- mouse models (EMBO J 1994, 13, 1189-1196). Such observations indicate that IL-6 plays an important role in osteogenesis and bone resorption particularly through the STAT3 phosphorylation.

In this regard, research has been actively carried out on the signal transduction system induced by IL-6 associated with metabolic bone diseases such as osteoporosis, inflammatory diseases, or autoimmune diseases. In particular, the inhibition of the IL-6-induced signal transduction system using anti-IL-6 receptor antibody (IL-6 R antibody) has been most well known. Specifically, a synovial cell growth inhibitor for rheumatoid arthritis remedy using IL-6 R antibody (WO Publication No. 96/011020), and treatment of plasmacytosis, hyperimmunoglobulinemia, anemia, nephritis, cachexia, rheumatoid arthritis, livestock-farmer disease, and mesangium proliferative nephritis induced by IL-6 products using anti-IL-6 R antibody (WO Publication No. 96/012503) is already known. Additionally, anti-IL-6 R antibody has been reported to be applicable to the treatment of multiple sclerosis, uveitis, chronic thyroiditis, and sensitized T-cell related diseases such as delayed hypersensitivity and atopic dermatitis (WO Publication No. 98/042377), treatment of systemic erythematosus, and treatment of Crohn's disease (WO Publication No. 99/047170), treatment of pancreatitis (WO Publication No. 00/010607), treatment of psoriasis (WO Publication No. 02/034292), and treatment of juvenile chronic arthritis (WO Publication No. 02/080969). However, in the case where the anti-IL-6 R antibody is introduced into a subject, it may have an epitope which may be perceived as a foreign protein by the subject, and this may be a problem of being subject to immunogenicity when used as a therapeutic agent. In order to solve such problem, numerous studies have been conducted to develop therapeutic agents mediated by IL-6 using small molecule compounds not recognized by immune systems instead of proteins.

Meanwhile, *Rosa rugosa Thunberg* is a perennial shrub of rose native, and contains rosamultin, quercetin, citric acid, malic acid, lycopene, tormentic acid, euscaphic acid, biaaborossol A, and other various components, while its flowers and fruits are used ornamentally and as a raw material for perfume or medicine. In particular, the initial flush of bloom in May is called *Rosa Rugosa Flos,* and is used in the treatment of hematemesis, elasticity, irregular menstruation, dysentery, *etc.,* and its fruits are used for fatigue recovery while its roots are used as a folk remedy for diabetes (Namba (1994) The encyclopedia of Wakan-yaku (traditional Sino-Japanese Medicines) which color pictures Vol (II). Hoikuaha, Tokyo, p. 118).

It has further been described that Rosa rugosa Thunberg flower extract could be utilized as anti-aging and whitening cosmetic ingredients (YU, HYE-SOO ET AL.: "The Study on Pharmacological Activation as Cosmetic Material of Rosa Rugosa Thunb. Flowers Extract", THE KOREAN JOURNAL OF MERIDIAN & ACUPOINT, vol. 31, no. 4, 27 December 2014 (2014-12-27), pages 188-194).

Recently, it has been reported that fruit flesh and a seed extract *of Rosa rugosa Thunberg* have an impact on cells related to osteogenesis ( 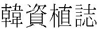 23(2):157∼164(2010) Korean J. Plant Res. (2010.04.16.). However, the fruit flesh and seed extract of *Rosa rugosa Thunberg* have cytotoxicity, and thus have a problem of inducing inhibitory activity of STAT3 luciferase induced by IL-6.

### Technical Problem

Under such circumstances, the present inventors endeavored to develop a natural substance that has a therapeutic effect on an IL-6-mediated disease aiming the STAT3 pathway which is activated by IL-6, and as a result, confirmed that the *Rosa rugosa Thunberg* flower water or ethanol extract effectively suppressed the signal transfer system mediated by IL-6 by suppressing the transcription activation and phosphorylation of STAT3, a transcription factor induced by IL-6, thereby completing the present invention.

### Technical Solution

An object of the present invention is to provide a pharmaceutical composition for use in preventing or treating an IL-6-mediated disease, comprising a *Rosa rugosa Thunberg* water or ethanol flower extract as an active ingredient, wherein the IL-6-mediated disease is osteoporosis, inflammatory bowel disease, or atopic dermatitis.

Still another object of the present invention is to provide a food composition for use in preventing or improving an IL-6-mediated disease, comprising a *Rosa rugosa Thunberg* flower water or ethanol extract as an active ingredient, wherein the IL-6-mediated disease is osteoporosis, inflammatory bowel disease, or atopic dermatitis.

Still another object of the present invention is to provide a feed composition for use in preventing or improving an IL-6-mediated disease, comprising a *Rosa rugosa Thunberg* flower water or ethanol extract as an active ingredient, wherein the IL-6-mediated disease is osteoporosis, inflammatory bowel disease, or atopic dermatitis

Still another object of the present invention is to provide a drinking water additive for use in preventing or improving an IL-6-mediated disease, comprising a *Rosa rugosa Thunberg* flower water or ethanol extract as an active ingredient, wherein the IL-6-mediated disease is osteoporosis, inflammatory bowel disease, or atopic dermatitis.

### Advantageous Effects

The present invention relates to a pharmaceutical composition for use in preventing or treating an IL-6-mediated disease, comprising a *Rosa rugosa Thunberg* flower water or ethanol extract as an active ingredient, wherein the IL-6-mediated disease is osteoporosis, inflammatory bowel disease, or atopic dermatitis. The present invention can be useful in the prevention or treatment of an IL-6-mediated disease, specifically, osteoporosis, inflammatory bowel disease, or atopic dermatitis, by inhibiting a signal transfer system induced by the IL-6 and differentiation of an osteoclast. Unlike an extract of other regions *of Rosa rugosa Thunberg,* the flower extract in particular has no cytotoxicity, and is advantageous in that it can be used in the treatment of said disease without any side effects, as it is derived from a natural substance that has long been used as a food product.

### Description of the Drawings

Fig. 1 shows the inhibitory activity of *Rosa rugosa Thunberg* flower ethanol extract, *Rosa rugosa Thunberg* flower water extract, and *Rosa rugosa Thunberg* fruit ethanol extract on the expression of IL-6-induced luciferase.
Fig. 2 shows the comparison of *Rosa rugosa Thunberg* flower ethanol extract, *Rosa rugosa Thunberg* flower water extract, and *Rosa rugosa Thunberg* fruit ethanol extract in terms of cytotoxicity.
Fig. 3 shows the inhibitory activity *of Rosa rugosa Thunberg* flower ethanol extract and *Rosa rugosa* fruit ethanol extract on the RANKL-induced osteoclast differentiation.
Fig. 4 shows the inhibitory effect of *Rosa rugosa Thunberg* flower extract on the osteoporosis induction using inflammatory osteoporosis mouse model.
Fig. 5 shows the inflammatory bowel disease-inhibitory effect of *Rosa rugosa Thunberg* flower ethanol extract and *Rosa rugosa Thunberg* flower water extract in a DSS-induced inflammatory bowel disease animal model.
Fig. 6 shows the inflammatory cytokine-inhibiting effect of *Rosa rugosa Thunberg* flower ethanol extract and *Rosa rugosa Thunberg* flower water extract in a DSS-induced inflammatory bowel disease animal model.
Fig. 7 shows the comparison *of Rosa rugosa Thunberg* flower ethanol extract and *Rosa rugosa Thunberg* flower water extract in terms of cytotoxicity.
Fig. 8 shows the inflammatory cytokine-inhibiting effect of *Rosa rugosa Thunberg* flower ethanol extract and *Rosa rugosa Thunberg* flower water extract.
Fig. 9 shows the inflammatory cytokine-inhibiting effect of *Rosa rugosa Thunberg* flower water extract.
Fig. 10 shows the inhibitory activity *of Rosa rugosa Thunberg* flower water extract on the STAT1 and NF-κB protein activation.

### Best Mode

An aspect of the present invention is to provide a pharmaceutical composition for use in preventing or treating an interleukin-6 (IL-6)-mediated disease, wherein the IL-6 mediated disease is osteoporosis, inflammatory bowel disease, or atopic dermatitis, comprising a *Rosa rugosa Thunberg* flower water or ethanol extract as an active ingredient.

As used herein, the term *"Rosa rugosa Thunberg"* is a perennial shrub of rose native, and contains rosamultin, quercetin, citric acid, malic acid, lycopene, tormentic acid, euscaphic acid, biaaborossol A, and other various components. It is known to be used ornamentally and as a raw material for perfume; in the treatment of hematemesis, elasticity, irregular menstruation, dysentery, and fatigue recovery; or as a folk remedy for diabetes. However, the *Rosa rugosa Thunberg* extract, particularly *Rosa rugosa Thunberg* flower extract, is not known for its therapeutic use for IL-6-mediated diseases, specifically, inflammatory bowel disease, metabolic bone diseases such as osteoporosis, or atopic dermatitis, which was first investigated by the present inventors. The *Rosa rugosa Thunberg* in the present invention may be purchased from commercially available sources or collected from or cultured in the wild.

As used herein, the term *"Rosa rugosa Thunberg* flower extract" means an extract obtained by extraction from a *Rosa rugosa Thunberg* flower. The present invention confirmed that among various regions *of Rosa rugosa Thunberg,* the *Rosa rugosa Thunberg* flower extract had a remarkable effect on the IL-6-induced inhibitory activity of STAT3 luciferase, specifically the excellence thereof in comparison with the *Rosa rugosa Thunberg* fruit extract.

The *Rosa rugosa Thunberg* flower may be extracted by using an about 2- to 20-fold, preferably, 3- to 5-fold volume of a polar solvent, for example, water; a C₁ to C₄ lower alcohol such as methanol, ethanol, butanol, *etc.*; or mixtures thereof in a ratio of approximately 1 :0.1 to 1: 10 at a temperature ranging from 20°C to 100°C, specifically room temperature, for a period ranging from about 12 hours to 4 days by hot water extraction, cold-immersion extraction, reflux cold extraction, sonication, *etc.,* but any method can be used without limitation as long as it is a method for extracting a substance having therapeutic activity on IL-6-mediated diseases. Specifically, it may be continuously extracted once to 5 times, and then filtered under reduced pressure, and the filtrate is concentrated using a rotary vacuum evaporator at 20°C to 100°C, more specifically at room temperature, to obtain a *Rosa rugosa Thunberg* flower extract that is solubilized in water, a lower alcohol, or a solvent mixture thereof. However, the method is not limited thereto as long as it has a therapeutic effect on IL-6-mediated diseases. The resultant includes all of a liquid extract, a diluted or concentrated liquid extract, a dry product obtained by drying the liquid extract, or a crude purified product, and a purified product thereof. The *Rosa rugosa Thunberg* flower extract may be extracted from natural, hybrid, and modified plants, and may be used solely, or bound with or in an appropriate combination with another pharmaceutical extract, fraction, or compound. In an exemplary embodiment of the present invention, ethanol or water was added to pulverized *Rosa rugosa Thunberg* flower and was extracted at room temperature (Example 1).

The *Rosa rugosa Thunberg* flower water or ethanol extract of the present invention can be effectively used in the prevention or treatment of IL-6-mediated diseases by inhibiting STAT3 activities.

As used herein, the term "IL-6-mediated diseases" generally refers to diseases caused by the activation of the signal transfer pathway due to IL-6, specifically the phosphorylation of STAT3 due to IL-6. The IL-6-mediated diseases include diseases that can be prevented or treated by the *Rosa rugosa Thunberg* flower extract and that are osteoporosis, inflammatory bowel disease, and atopic dermatitis. Specifically, IL-6, bound to a receptor such as gp130 and gp80, brings about phosphorylation of STAT3 protein, and the thus-activated STAT3 protein, by expressing a target gene in the nucleus, may cause onset or progress of a metabolic bone disease, inflammatory bowel disease, or atopic dermatitis.

As used herein, the term "metabolic bone diseases" includes osteoporosis by excessive osteoclastic bone resorption, rheumatoid arthritis, periodontal disease, Paget's disease, and diseases caused by bone destruction resulting from pathological bone diseases such as metastatic cancers; however, any disease can be included without limitation as long as it is a bone disease caused by the signal transfer pathway activated by IL-6. Specifically, the term "osteoporosis" refers to a condition in which the bone strength weakens due to loss of the bone mass and qualitative changes, thereby leading to a greater risk of bone fractures. Bones protect various internal organs and store calcium and other materials vital for the body. Bones also maintain a balance between osteoclasts facilitating the breakdown of bones in bone tissues and osteoblasts facilitating bone formation and thus homeostasis. Osteoporosis is caused and progressed by imbalance between the activity of the two cells and consequent excessive bone breakdown. As an important molecule in osteoporosis, receptor activator of nuclear factor kappa-B ligand (RANKL), bound to its receptor, promotes osteoclast differentiation by activating various transcription factors; in particular, IL-6 is known to promote osteoclast differentiation mediated by RANKL (Kim K, et al., J Immunol. 2007 May 1;178(9):5588-94.). As such, any material capable of inhibiting signal transfer activated by IL-6 and inhibiting osteoclast differentiation to decrease bone breakdown due to the osteoclasts can be used as a therapeutic agent for osteoporosis. The *Rosa rugosa Thunberg* flower water or ethanol extract of the present invention has been confirmed to be able to effectively inhibit the osteoclast differentiation, and thus can be useful in the prevention or treatment of osteoporosis.

As used herein, the term "inflammatory bowel disease (IBD)" refers to a disease which occurs when CD4+ T lymphocytes in the intestinal mucous membrane cells are abnormally activated due to foreign microorganisms and antigen-presenting cells, leading to the continuous expression of inflammatory cytokines (TNF-α, IL-6, IL-12, IL-17, IL-23, and IL-27). In the present invention, the prophylactic or therapeutic effect of IBD was confirmed in an animal model having an IBD induced by DSS treatment.

As used herein, the term "atopic dermatitis" is a type of skin disease, accompanied by wheal, eczema, and itching. It is known that environmental and genetic factors are involved in the pathogenesis of the atopic dermatitis, although the precise mechanism thereof has not been investigated. The atopic dermatitis recognizes proteins derived from keratic cells or epidotheliocytes as antigens and may cause a symptom of an immune response, but is not limited thereto. According to existing reports, atopic dermatitis is characterized in possessing autoantibodies against CD28, where the anti-CD28 autoantibodies not only cause the activation of T lymphocytes but also play a role as a distinctive feature which enables differentiation from other inflammatory skin diseases (Clin Exp Immunol. 2006 Nov;146(2):262-9.). Recently, atopic dermatitis has frequently occurred not only in infants and children but also in adults, and accordingly, while its prevention and treatment have become an important social problem, the causes giving rise to atopic dermatitis are clear. Furthermore, the efficacy of existing steroid formulations, *etc.* is not outstanding and there is a problem of accompanying side effects. In this regard, there has been a demand for the development of a safe drug for treating atopy which can be used in infants and children while having excellent efficacy. The composition of the present invention is derived from a natural substance and is advantageous in that compared to steroids, it is safer for application to infants and children. As such, it has been confirmed in the present invention that by inhibiting the IL-6-mediated pathway using the composition and relieving edema due to the IL-6-mediated signal transfer pathway and other various inflammatory symptoms associated with atopic dermatitis using the composition, the atopic dermatitis can be effectively treated.

The metabolic bone diseases, inflammatory bowel disease, and atopic dermatiti may be induced by IL-6.

The *Rosa rugosa Thunberg* flower water or ethanol extract inhibits the differentiation of osteoclasts and thus can be effectively used in the prevention or treatment of IL-6 mediated diseases. Specifically, the *Rosa rugosa Thunberg* flower water or ethanol extract can inhibit the osteoclast differentiation induced by RANKL.

As used herein, the term "osteoclasts" refers to hemopoietic cell-derived cells which are involved in bone resorption and play an important role in bone regeneration. Bone resorption due to osteoclasts consists of several steps including: differentiation into an osteoclast precursor; formation of multinucleated osteoclasts due to fusion; and activation of matured osteoclasts for bone resorption. The matured osteoclast-like multinucleated cells (OCL) maintain a normal actin cytoskeleton, undulated membrane and acidic conditions.

As used herein, the term "RANKL", as an abbreviation for "receptor activator of nuclear factor kappa-B ligand", is also known as tumor necrosis factor ligand superfamily member 11 (TNFSF11), TNF-related activation-induced cytokine (TRANCE), osteoprotegerinligand (OPGL), and osteoclast differentiation factor (ODF). RANKL is a human endogenous protein encoded by TNFSF11 gene, and is known as a type-II membrane protein and a member of the tumor necrosis factor (TNF) superfamily.

As used herein, the term "prevention" refers to any behavior resulting in suppression or delay of the onset of the IL-6-mediated diseases by the administration of the composition, and the term "treatment" refers to any action resulting in improvement in symptoms of the IL-6-mediated diseases or the beneficial alteration by the administration of the composition.

The pharmaceutical composition comprising the *Rosa rugosa Thunberg* flower water or ethanol extract may further include appropriate carriers, excipients, and diluents that are conventionally used in the preparation of a pharmaceutical composition. Specifically, the pharmaceutical composition can be formulated into oral preparations such as powder, granule, tablet, capsule, suspension, emulsion, syrup, aerosol, *etc.;* preparations for external use; suppository; or sterile solution for injection. The content of the *Rosa rugosa Thunberg* flower extract is not particularly limited, but may be 0.0001 wt% to 10 wt%, more specifically 0.001 wt% to 1 wt%, based on the total weight of the composition.

The pharmaceutical composition may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil as the carriers, excipients, and diluents. When preparing the composition into a formulation, a generally used diluent or excipient such as filler, expander, binder, humectant, disintegrant, or surfactant may be used. A solid oral formulation includes a tablet, a pill, a powder, a granule, a capsule, *etc.* Such solid formulations may include at least one excipient, for example, starch, calcium carbonate, sucrose or lactose, gelatin, *etc.* including the *Rosa rugosa Thunberg* flower extract. A lubricant, such as magnesium stearate and talc, may be used in addition to a simple excipient. A liquid oral formulation includes a suspension, a solution, an emulsion, syrup, *etc.,* and may include a simple diluent such as water, liquid paraffin; and various excipients, e.g., a humectant; a sweetening agent; an odorant; a preservative, *etc.* A parenteral formulation includes a sterile aqueous solution, a non-aqueous solution, a suspension, an emulsion, a lyophilized formulation, and a suppository. A non-aqueous solvent or a suspending agent includes propylene glycol, polyethylene glycol, vegetable oil such as olive oil, or injectable ester such as ethyl oleate. Bases for the suppository may be witepsol, macrogol, Tween 61, cacao butter, Laurin, glycerogelatine, *etc.*

The composition may be administered in a pharmaceutically effective amount.

As used herein, the term "pharmaceutically effective amount" refers to an amount sufficient to treat or prevent a disease at a reasonable risk/benefit ratio applicable to any medical treatment. An effective dosage level of the composition may be determined depending on factors including the type of a subject and severity of the disease, patient's age, gender, type of the disease, activity of the drug, patient's sensitivity to the drug, administration time, route of administration, excretion rate, duration of treatment, drugs used in combination, and other factors known in the medical field. The composition may be administered individually or in combination with other therapeutic agents, and may be administered sequentially or simultaneously with conventional therapeutic agents. The composition may be administered in single or multiple dosage forms. It is important to administer the composition in a minimum amount that can exhibit a maximum effect without causing side effects, in consideration of all of the above-described factors, and the amount can be easily determined by one of ordinary skill in the art. A preferable dosage of the composition varies depending on the condition and body weight of patient, severity of disease, drug form, and route and period of administration; however, for a preferred effect, the *Rosa rugosa Thunberg* flower water or ethanol extract may be administered daily in an amount of 0.0001 mg/kg to 100 mg/kg, preferably 0.001 mg/kg to 100 mg/kg, and more specifically 0.001 mg/kg to 500 mg/kg. The administration can be carried out once or several times a day.

The composition may be administered to mouse, livestock, humans, and other mammals via a variety of routes. There is no particular limitation on the administration routes; for example, the composition can be administered orally, rectally, or by intravenous, intramuscular, subcutaneous, intra-uterine, or intracerebroventricular injection.

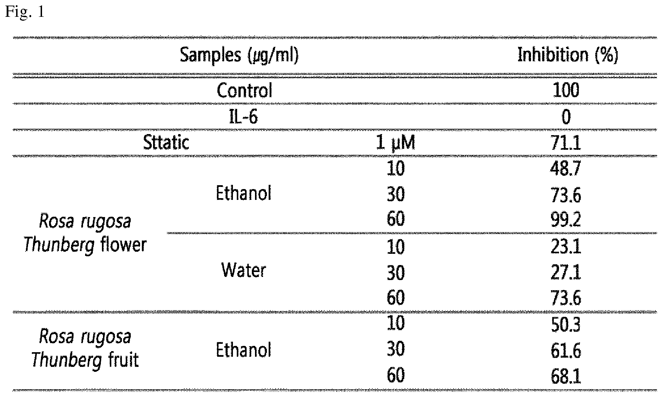

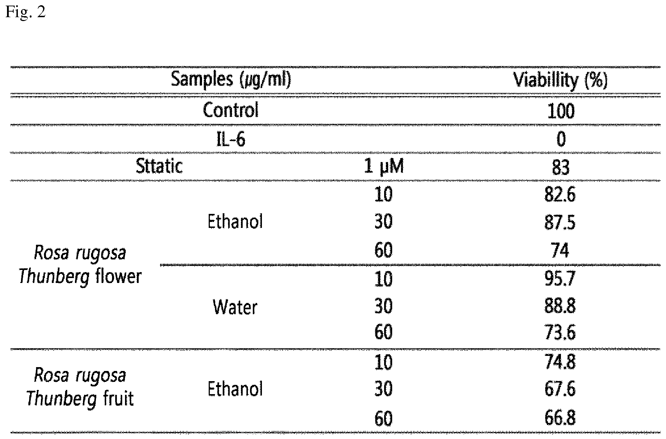

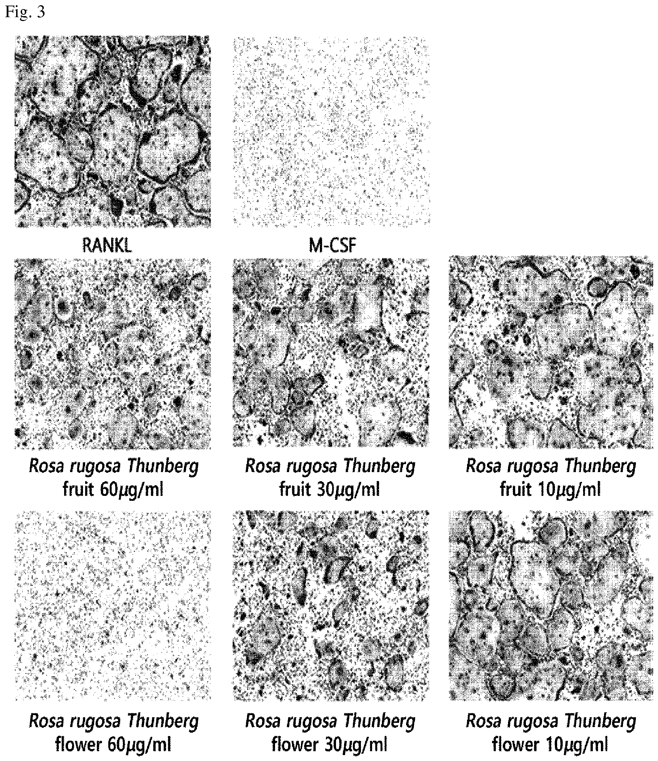

In a specific exemplary embodiment of the present invention, it was confirmed that flower ethanol extract and water extract of the *Rosa rugosa Thunberg,* by effectively inhibiting the activity of STAT3 luciferase induced by IL-6, have remarkably excellent activity compared to the *Rosa rugosa Thunberg* fruit extract ethanol (Fig. 1), and the cytotoxicity exhibited in the *Rosa rugosa Thunberg* flower extract is remarkably lower than in the *Rosa rugosa Thunberg* fruit extract (Fig. 2). Additionally, as a result of comparing the effect of the *Rosa rugosa Thunberg* flower extract and fruit extract on the osteoclast differentiation, the *Rosa rugosa Thunberg* flower ethanol showed an excellent inhibitory effect on the osteoclast differentiation induced by RANKL extract when its concentration was 60 µg/mL and 30 µg/mL, whereas the *Rosa rugosa Thunberg* fruit extract showed no significant effect (Fig. 3).

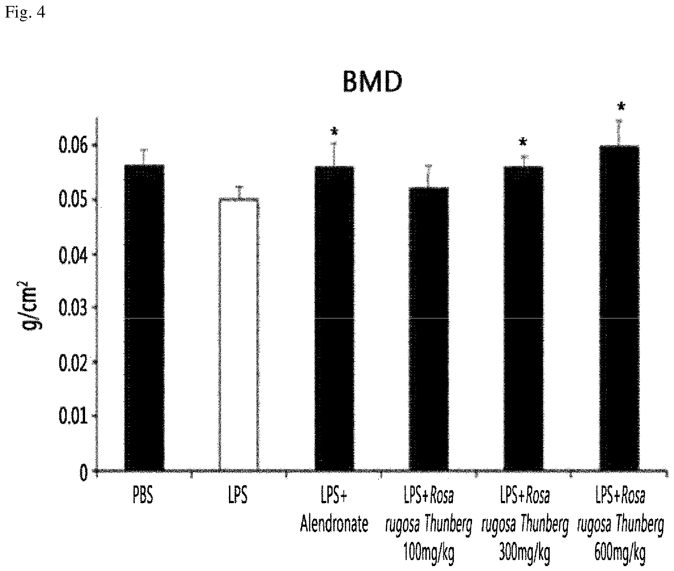

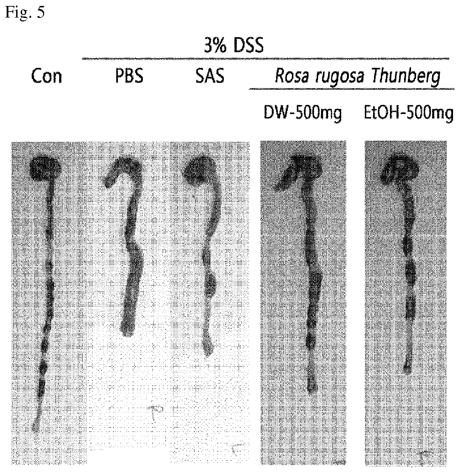

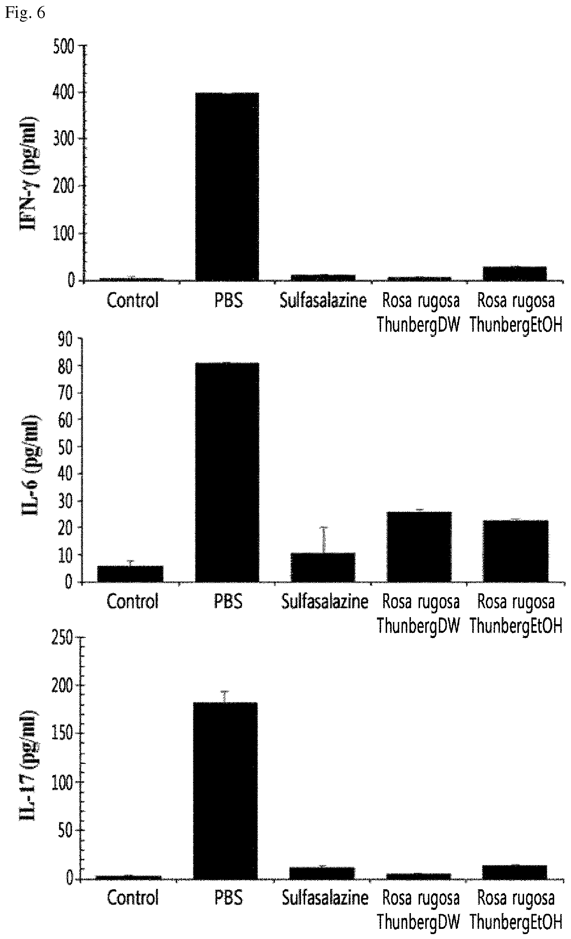

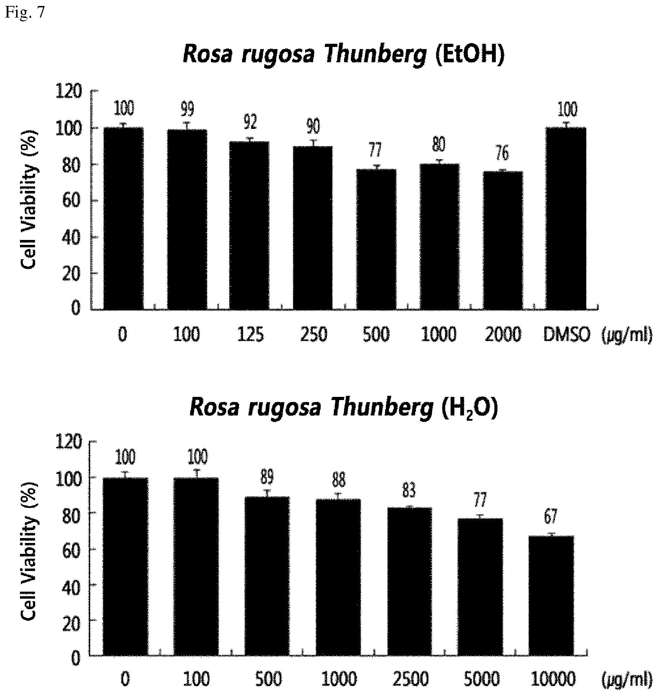

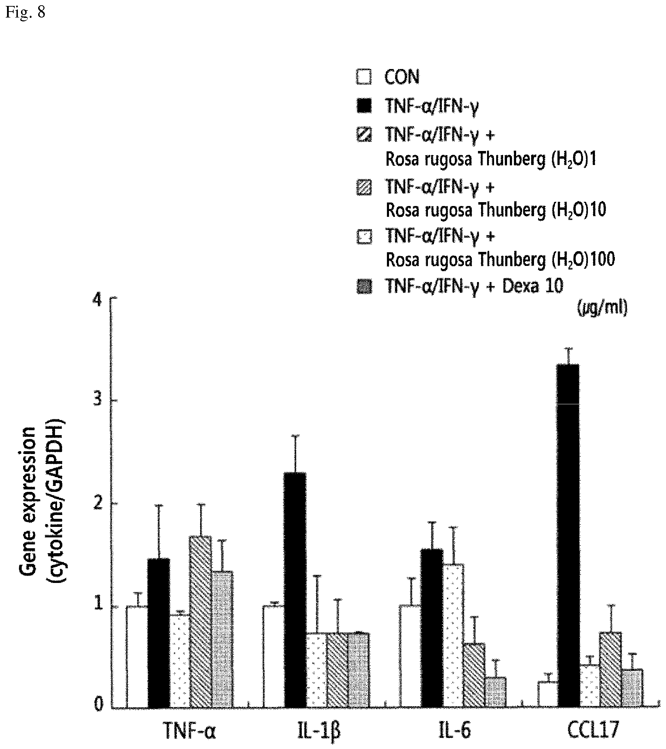

Additionally, the effect of the *Rosa rugosa Thunberg* flower extract on the IL-6-mediated diseases was measured; specifically, when the *Rosa rugosa Thunberg* flower extract was administered in a mouse model having inflammatory osteoporosis, lowered bone mineral density and bone mineral contents in the LPS-induced group were significantly recovered (Fig. 4); and when administered (500 mg/kg) in a mouse model having inflammatory bowel disease, IBD symptoms induced by DSS treatment were treated and inflammatory cytokines (IL-6, IL-17, and IFN-g) were effectively inhibited (Figs. 5 and 6). In the case of atopic dermatitis, the *Rosa rugosa Thunberg* flower extract had no toxic effects on HaCaT cells even when its concentration was 1000 µg/mL, and showed the inhibitory effect against inflammatory cytokines TNF-α, IL-1β, IL-6, and CCL17 (Figs. 7 and 8).

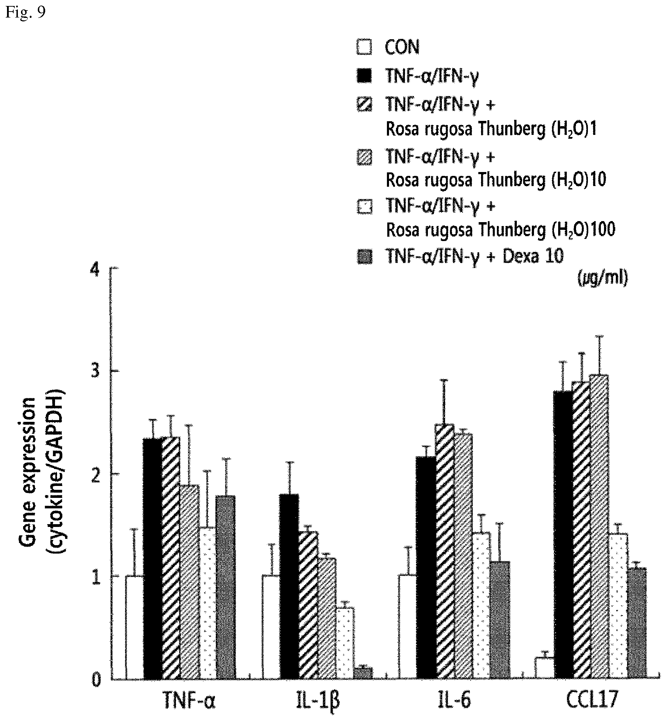

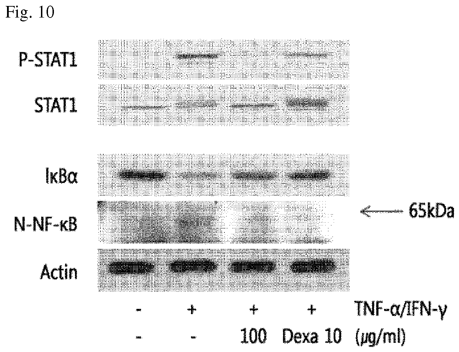

In particular, the *Rosa rugosa Thunberg* water extract had a highly excellent inflammatory cytokine-inhibiting effect compared to the ethanol extract, where the inhibiting effect was concentration-dependent (Fig. 9). It was also confirmed that the *Rosa rugosa Thunberg* water extract at the concentration of 100 µg/mL showed an effect of decreasing NF-κB by inhibition of IκBα differentiation and STAT1 phosphorylation activated by TNF-α/INF-γ (Fig. 10). The pharmaceutical composition may be administered parenterally, subcutaneously, intraperitoneally, intrapulmonarily, or intranasally. For local immunosuppressive therapy, the composition may, if necessary, be administered using a suitable method, including intralesional administration. A preferable dosage of the composition may vary depending on the condition and body weight of patient, severity of disease, drug form, and route and period of administration, but may be appropriately selected by one of ordinary skill in the art.

Still another aspect of the present invention provides a food composition for use in preventing or improving an IL-6-mediated disease, comprising a *Rosa rugosa Thunberg* flower water or ethanol extract as an active ingredient, wherein the IL-6-mediated disease is osteoporosis, inflammatory bowel disease, or atopic dermatitis.

The *Rosa rugosa Thunberg* flower extract and IL-6-mediated diseases are the same as previously described.

Specifically, the extract can be added to the food composition for the purpose of preventing or improving an IL-6-mediated disease, specifically, improving a bone metabolic disease, inflammatory bowel disease, or atopic dermatitis.

In the case where the extract is used as a food additive, the *Rosa rugosa Thunberg* flower water or ethanol extract may be added to foods, as it is or together with other foods or food ingredients, and properly used according to a conventional method. An amount of the active ingredients may be appropriately determined according to the purpose of use (prophylactic, health, or therapeutic treatment), and a sitologically acceptable food additive may be further included in the composition of the present invention. Because the composition has an extract derived from a natural substance as an active ingredient and thus has no safety issue, there is no limitation on the amount of the active ingredients that are mixed. Meanwhile, the food composition may include any conventional food, and can be interchangeably used with commonly known terms in the art such as a functional food, health functional food, *etc..*

As used herein, the term "functional food" refers to a food prepared or processed using raw materials or ingredients that have useful functional properties for the human body as stipulated by Korean Law 6727 regarding health functional foods, and the term "functional" refers to adjusting nutrients for structures and functions of the human body and obtaining useful effects for health use such as physiological action, *etc.* Additionally, the term "health functional foods" refers to a food having a particular ingredient as a raw material for the purpose of health supplementation or prepared or processed by a method of extracting, concentrating, purifying, mixing, *etc.* a particular ingredient contained in a food raw material; and refers to a food that is designed and processed to exhibit a body-modulating function such as body defense, regulation of biorhythm, prevention of and recovery from disease, *etc.* The health functional food composition can perform the functions of preventing and recovering from diseases.

There is no limitation on the type of the foods in which the composition can be used. Additionally, the composition containing the *Rosa rugosa Thunberg* flower extract as an active ingredient can be prepared with an appropriate supplemental ingredient and additive known in the art, which are selected by one of ordinary skill in the art. Examples of the foods that can be added include meat, sausages, bread, chocolate, candies, snacks, pizza, ramen noodles, other noodles, gums, dairy products such as ice cream, various soups, beverages, tea, energy drinks, alcohol, vitamin complex, *etc.* Concentrates, tea, jellies, and juice which contain the extract as the main ingredient can also be added in preparation of the health functional foods.

Additionally, the foods applicable to the present invention may include specialized nutritional foods (*e.g.,* milk formulas, baby/infant food, *etc.*), meat products, fish meat products, tofu, jelled foods, noodles (*e.g.,* ramen noodles, noodles, *etc.*), dietary supplements, seasonings (*e.g.,* soy sauce, soybean paste, red pepper paste, mixed paste, *etc.*), sauces, confectionery (*e.g.,* snacks), milk products (*e.g.,* fermented milk, cheese, *etc.*), other processed foods, kimchi, pickled foods (*e.g.,* various kinds of kimchi, pickled vegetables, *etc.*), beverages (*e.g.,* fruit drinks, vegetable beverages, soy bean milk, fermented beverages, *etc.*), natural seasonings (*e.g.,* ramen soup base, *etc.*), *etc.,* and all other kinds of foods.

In the case where the health functional food composition is used in the form of a drink, as a conventional drink, it may further contain various sweetening agents, flavoring agents, natural carbohydrates, *etc.* as additional ingredients. In addition thereto, the health functional food composition may contain nutrients, vitamins, electrolytes, flavoring agents, colorants, pectic acid and its salt, alginic acid and its salt, organic acids, protective colloidal thickeners, pH-adjusting agents, stabilizers, preservatives, glycerin, alcohols, carbonating agents used in a carbonated drink, *etc.* The health functional food composition may also contain fruit flesh that may be used for preparing natural fruit juice, fruit juice drinks, and vegetable drinks.

The *Rosa rugosa Thunberg* flower extract and IL-6-mediated diseases are the same as previously described.

Still another aspect of the present invention provides a feed composition for use in preventing or improving an IL-6-mediated disease, wherein the IL-6-mediated disease is osteoporosis, inflammatory bowel disease, or atopic dermatitis, comprising a *Rosa rugosa Thunberg* flower water or ethanol extract as an active ingredient; and a drinking water additive for use in 2. preventing or improving an IL-6-mediated disease, comprising a *Rosa rugosa Thunberg* flower water or ethanol extract as an active ingredient, wherein the IL-6-mediated disease is osteroporosis, inflammatory bowel disease, or atopic dermatitis.

The prevention or improvement of the IL-6-mediated diseases of the *Rosa rugosa Thunberg* flower extract is as described above.

For the purpose of the present invention, the *Rosa rugosa Thunberg* flower water or ethanol extract can be used for the improvement of the IL-6-mediated diseases, wherein the IL-6-mediated disease is osteoporosis, inflammatory bowel disease, or atopic dermatitis, and thus can be included as an additive of a feed or drinking water.

As used herein, the term "feed composition" generally refers to any substance added to a feed in an infinitesimal amount for a nutritional or certain purpose, and in the present invention, refers to a substance added for the purpose of improving IL-6-mediated diseases. The term "drinking water additive" generally refers to any material added to drinking water in an infinitesimal amount for a nutritional or certain purpose for animals, and in the present invention, refers to a substance added for the purpose of preventing or improving IL-6-mediated diseases.

The *Rosa rugosa Thunberg* flower water or ethanol extract can be added to the feed composition as a feed additive, and the feed refers to all foods that are supplied to animals.

As used herein, the term "animal" refers to all livestock and pets; specifically companion pet animals (*e.g*., dogs, cats, *etc.*)*,* pigs, cattle, *etc.,* but is not limited thereto. The feed composition or drinking water additive may further contain a binder, lubricating agent, preservative, *etc.* for prevention of quality deterioration thereof; and an amino acid, vitamin, enzyme, probiotic, flavoring agent, non-protein nitrogen compound, silicate salt, buffering agent, coloring agent, extracting agent, oligosaccharides, *etc.* for increase in utility thereof; as well as other feed mixture, *etc.,* but is not limited thereto.

### Mode for Invention

Hereinbelow, the present invention will be described in detail with accompanying exemplary embodiments. However, the exemplary embodiments disclosed herein are only for illustrative purposes and should not be construed as limiting the scope of the present invention.

### Example 1: Preparation of extract of each region of Rosa rugosa Thunberg

After thoroughly washing with water and drying under shade, a flower and a fruit of the *Rosa rugosa Thunberg* were ground into powder using a Waring blender. 500 g of the ground *Rosa rugosa Thunberg* flower and fruit were added to 5 L of ethanol and extracted at 70°C for 10 hours. The extract was filtered under reduced pressure using filter paper (Whatman, USA), and the filtrate was concentrated by removing ethanol using a rotational vacuum concentrator at room temperature to obtain an ethanol extract of the *Rosa rugosa Thunberg* flower and fruit. Additionally, 500 g of ground the *Rosa rugosa Thunberg* flower was added to 5 L of distilled water and extracted at room temperature for 24 hours. The extract was filtered under reduced pressure using filter paper (Whatman, USA), and the filtrate was concentrated by removing water using a rotational vacuum concentrator at room temperature to obtain a water extract of the *Rosa rugosa Thunberg* flower and fruit.

### Example 2: The IL-6 induced inhibitory effect of Rosa rugosa Thunberg extracts on STATS transcription activity

The following experiments were performed in order to measure the IL-6-induced inhibitory effect of the *Rosa rugosa Thunberg* extract of each on STAT3 transcription activity.

### 2-1. Preparation of transformant

Hep3B cells (ATCC HB-8064) were transfected with pStat3-Luc and pcDNA3.1 (+) (Clontech laboratories, Palo Alto, CA) using lipofectamin plus (Invitrogen, Carlsbad, CA, USA). Two days after the transfection, the transfected cells were treated with hygromycin at a concentration of 100 µg/mL to obtain clones which stably express luciferase. The stable expression of luciferase on the clones was confirmed via luciferase assay.

### 2-2. IL-6-reactive STATS reporter gene assay

The transfected cells were cultured in DMEM (GIBCO 119950965) under serum starvation and treated for 1 hour as shown below, followed by 12 hours of culturing with 10 ng/mL IL-6 (R&D system, USA) added.
1: Negative control group (non-treated group)
2: Positive control group (IL-6 10 ng/mL)
3: Extracts (10 µg/mL, 30 µg/mL, 60 µg/mL)

The reacted cells were washed with PBS, dissolved in 50 µL of a buffer solution (luciferase assay system, Promega, USA), and stirred for 20 minutes. 30 µL to 100 µL of luciferase substrate (luciferase assay system, Promega, USA) was added, and the level of color development was measured within 5 minutes using a luminometer (EG&G BERTHOLD, USA). Oleanolic acid acetate (OAA), a compound which has an IL-6-inhibiting effect, was used as the positive control group (Rheumatology, 2014, 53(1), 56-64). The assay result revealed that the *Rosa rugosa Thunberg* ethanol and water extracts inhibited the activity of STAT3 luciferase in a concentration-dependent manner. Specifically, the *Rosa rugosa Thunberg* flower extract showed a significant effect, i.e., inhibited the luciferase activity at the concentration of 60 µg/mL by 97.9%; whereas the *Rosa rugosa Thunberg* fruit extract showed a less significant effect compared to the *Rosa rugosa Thunberg* flower extract (Fig. 1).

The above results confirm that the *Rosa rugosa Thunberg* flower extract, by significantly inhibiting the transcription activity of the STAT3 luciferase that is an important mediator in the signal transfer system of inflammatory diseases induced by IL-6, has a significant effect on the prevention or treatment of the diseases mediated by IL-6.

### Example 3: Confirmation of cytotoxic effect of the Rosa rugosa Thunberg flower extract

The transfected cells cultured in DMEM (GIBCO 119950965) under serum starvation were seeded in a 96-well plate, and treated for 24 hours as shown below.
1: Negative control group (non-treated group)
2: Positive control group (IL-6 10 ng/mL)
3: Extracts (10 µg/mL, 30 µg/mL, 60 µg/mL)

After the 24-hour treatment, 20 µL of MTT solution (5 mg/mL) was added to each well and cultured for 4 hours. The supernatant was then removed, and formazan crystal, formed by the MTT reaction, was dissolved by adding 100 µL of DMSO. The level of color development was measured by absorbance at 570 nm using a plate reader (Model680, BIO-RAD). The assay result revealed that in the *Rosa rugosa Thunberg* flower ethanol extract, the cell viability was observed to be 80% or higher even when the concentration of the flower extract was 60 µg/mL, indicating no significant cytotoxicity; in contrast, not only was the cytotoxicity of the *Rosa rugosa Thunberg* fruit ethanol extract generally high, but also the cell viability of 66.8% was measured when its concentration was 60 µg/mL, indicating that the fruit cytotoxicity was much higher than the flower extract (Fig. 2). This showed that the *Rosa rugosa Thunberg* flower extract is safer than the *Rosa rugosa Thunberg* fruit extract, and in view with the results of the *Rosa rugosa Thunberg* fruit extract in Example 2, the fact that *Rosa rugosa Thunberg* fruit extract has the activity of inhibiting STAT3 luciferase induced by IL-6 could be predicted to be induced by cytotoxicity.

### Example 4: Inhibitory effect of Rosa rugosa Thunberg flower extract on RANKL-induced osteoclast differentiation

The inhibition of the transcription activity of the STAT3 luciferase induced by IL-6 is shown in Example 2. This example is to confirm the direct therapeutic efficacy of the *Rosa rugosa Thunberg* flower ethanol extract on osteoporosis.

The following assay was conducted to measure the inhibitory effect of the *Rosa rugosa Thunberg* flower ethanol extract on osteoclast differentiation. Femur and tibia were removed from 5-week-old mice, and the bone cavities were washed using a 1 cc syringe to obtain bone marrow cells. The isolated bone marrow cells were cultured in a-minimum essential medium (a-MEM) containing 10% FBS, antibiotic, and M-CSF (30 ng/mL) for 3 days. After 3 days, the adherent cells were used as bone marrow macrophage (BMM). The bone marrow macrophages were cultured by adding M-CSF (30 ng/mL) and RANKL (50 ng/mL) and treated with the *Rosa rugosa Thunberg* flower ethanol extract at each concentration of 60 µg/mL, 30 µg/mL, and 10 µg/mL. After 4 days, the cultured cells were stained with TRAP solution (Sigma Aldrich, USA), and red stained cells were determined to be differentiated osteoclasts. As a result, the *Rosa rugosa Thunberg* flower ethanol extract at the concentrations of 60 µg/mL and 30 µg/mL showed the excellent effect of inhibiting RANKL-induced osteoclast differentiation, whereas the inhibitory effect of the *Rosa rugosa Thunberg* fruit ethanol extract was not noticeable (Fig. 3).

Such results confirm that the effect of the *Rosa rugosa Thunberg* fruit extract on osteoporosis is not significant, while the *Rosa rugosa Thunberg* flower extract has an excellent therapeutic effect on osteoporosis.

### Example 5: Inhibitory effect of the Rosa rugosa Thunberg flower ethanol extract against osteoporosis induction using mouse model having inflammatory osteoporosis

To measure the inhibitory effect of the *Rosa rugosa Thunberg* flower ethanol extract on osteoporosis induction, a mouse model having inflammatory osteoporosis was used. 7-week-old C57BL/6 female mice were subcutaneously injected on the skull with 25 mg/kg LPS once a week, three times in total. The experimental group was divided into 6 groups of PBS, LPS, LPS + alendronate (2 mg/kg), LPS + the *Rosa rugosa Thunberg* flower ethanol extract (100 mg/kg, 30 mg/kg, and 600 mg/kg). While having the PBS group as the control group with no LPS administration, and the remaining groups as the LPS-induced osteoporosis group, the positive control group was administered with alendronate, a drug for osteoporosis, and the *Rosa rugosa Thunberg* flower test group was administered with each drug once daily for 4 weeks total.

After 4 weeks of the oral administration, the bone mineral density (BMD) and bone mineral contents (BMC) were measured via Dual energy X-ray analysis (DEXA) to evaluate the therapeutic effect of the *Rosa rugosa Thunberg* flower ethanol extract on osteoporosis. As a result, the lowered BMD and BMC in the LPS-induced group were significantly recovered in the group administered with the osteoporosis drug, and those in the group administered with the *Rosa rugosa Thunberg* flower ethanol extract (300 mg/kg and 600 mg/kg) were also significantly recovered (when compared to the LPS group, *p<0.05) (Fig. 4).

### Example 6: Inhibitory effect of the Rosa rugosa Thunberg flower ethanol and water extracts against inflammatory bowel disease induction using mouse model having inflammatory bowel disease

To measure the inhibitory effect of the *Rosa rugosa Thunberg* flower ethanol and water extracts on the inflammatory bowel disease, a mouse model having inflammatory bowel disease was used. 4-week-old ICR male mice (Orient Bio Inc., Korea) were adapted to the dark-light cycle for 12 hours, and further had 7 days of adaption before the initiation of the experiment. For all groups excluding the control group, a 3% DSS solution was prepared by adding DSS to distilled water and administered *ad libitum* for 14 days to prepare animal models of IBD. From 2 days before the addition of the 3% DSS, the control group and 3% DSS group, sulfasalazine (SS) group, and the group of the *Rosa rugosa Thunberg* flower ethanol and water extracts were orally administered PBS, 50 mg/kg sulfasalazine, and 500 mg/kg *Rosa rugosa Thunberg* flower ethanol and water extracts daily, respectively. After 16 days, the mice were sacrificed by dislocation of cervical vertebra, and their blood vessel, spleen, and intestines were incised. The length of the intestines was measured from the appendix to the rectum, and the colon and the rectum were used for histological analysis.

As a result, it was confirmed that the test group treated with 500 mg/kg *Rosa rugosa Thunberg* flower ethanol and water extracts showed an excellent effect of treating IBD symptoms induced by the DSS treatment (Fig. 5), and the inflammatory cytokines (IL-6, IL-17, and IFN-γ) induced by the DSS treatment were effectively inhibited in the experimental group treated with the *Rosa rugosa Thunberg* flower ethanol and water extracts (Fig. 6).

### Example 7: Efficacy verification assay of the Rosa rugosa Thunberg flower ethanol and water extracts

### 7-1: Anti-atopic effect of the Rosa rugosa Thunberg flower ethanol and water extracts using HaCaT cells

The anti-atopic effect of the *Rosa rugosa Thunberg* flower ethanol and water extracts was measured using human keratinocyte cell line, HaCaT. The HaCaT cells were cultured in Dulbecco's modified Eagle Medium (DMEM) containing 10% heat-inactivated FBS and 1% penicillin-streptomycin at 37°C under 5% CO₂. The viability of the cultured cells was measured using an MTT [3-(4,5-dimethylthiazole-2-yl)-2,5-diphenyl-tetrazolium bromide] reduction method. The MTT solution produces formazan due to dehydrogenases of mitochondria present in living cells, indicating the cell viability. To measure the cytotoxicity, HaCaT cells were cultured in a 96-well plate at a concentration of 5× 10⁴ cells/well at 37°C, and treated with 100 µg/mL to 2000 µg/mL *Rosa rugosa Thunberg* flower ethanol extract and 100 µg/mL to 10000 µg/mL *Rosa rugosa Thunberg* flower water extract followed by 24 hours of culturing. After 24 hours, 20 µL of MTT solution was added to each well and further cultured for 2 hours. The supernatant was then removed, and absorbance was measured at 570 nm by adding 100 µL dimethylsulfoxide. The *Rosa rugosa Thunberg* flower ethanol and water extracts did not show cytotoxicity at a concentration of 500 µg/mL or below in the HaCaT cells (Fig. 7).

To determine cytokines related to atopic dermatitis, the cultured HaCaT cells were treated with TNF-α/INF-γ. After inducing atopy-like conditions, each of 1000 µg/mL *Rosa rugosa Thunberg* flower ethanol and water extracts was treated and cultured followed by extracting RNA from the cells. 10 µg/mL dexamethasone, which has an anti-atopy effect, was used as the positive control group. The entire RNA was isolated from the cells using an RNA isolation kit (RNAiso Plus reagent, Takarabio), whose absorbance was then measured and quantified, and cDNA was synthesized using 2 µg of RNA. Each tube was added with 2 µL of cDNA, 1 µL of sense and antisense primer solutions (0.4 µM), 12.5 µL of SYBR Premix Ex Taq (Takarabio), and 9.5 µL of dH₂O to a final volume of 25 µL. The expression levels of TNF-α, IL-1β, IL-6, and CCL17 mRNAs were measured via real-time PCR using TP850 software. TNF-α, IL-1β, IL-6, and CCL17 cytokines were confirmed to be expressed when atopy was induced in the HaCaT cells.

As a result, at the concentration of 1000 µg/mL, the *Rosa rugosa Thunberg* flower water extract showed a better cytokine-inhibiting effect compared to the ethanol extract (Fig. 8). Further, as a consequence of treating the *Rosa rugosa Thunberg* flower water extract in the concentrations of 1 µg/mL, 10 µg/mL, and 100 µg/mL, the amount of cytokine expression was inhibited in a concentration-dependent manner (Fig. 9).

### 7-2: Mechanism of signal transfer involved in the inhibition of inflammation-inducing cytokine expression of Rosa rugosa Thunberg water extract

In order to investigate the signal transfer mechanism of the *Rosa rugosa Thunberg* flower water extract involved in the inhibition of inflammation-inducing cytokines, the activation of NF-κB, an important signal transfer factor that regulates the transcription of inflammatory cytokines and STAT1 phosphorylation, was measured. An inactive form of NF-κB binds to an endogenous inhibitory protein IκBα and maintains its inactive form. The degradation effect on the IκBα that is released and transferred to the nucleus and acts as a transcription factor upon activation by phosphorylation and degradation was also measured. The HaCaT cells were dissolved in an extraction buffer (PBS solution containing 0.5% triton X-100, 1 mM Na₃VO₄, etc.) containing 0.5 mM phenylmethanesulfonyl fluoride (Sigma, Cat No. P7626) and 5 µg/mL leupeptin (Sigma, Cat No. L2884), and sonicated to obtain DNA fragments. The protein amount was measured using Bio-Rad protein assay kit (Cat No. 500-0002) based on bovine serum albumin. Electrophoresis was performed in 8%(w/v) to 12%(w/v) polyacrylamide gel containing 0.1% SDS for the total protein amount of 10 µg to 50 µg, and the protein present in the gel was transferred to a nitrocellulose (NC) filter using an electroblotting method. To prevent non-specific bindings, the NC filter was placed in tris-buffered saline-tween (TBS-tween, Sigma) containing 5% dried milk and soaked at room temperature for 1 hour. The filter was added to the TBS-tween solution containing an antibody to a target protein and allowed to sit at 4°C for 12 hours, and was then labeled as secondary antibody labeled with horseradish peroxidase (HRP, Sigma, Cat No. P0889). Enhanced chemiluminescence (ECL, Thermo scientific, Cat No. 34080) was used to measure the bands. The results revealed that the *Rosa rugosa Thunberg* flower water extract inhibits the phosphorylation of STAT1 activated by TNF-α/INF-γ and the differentiation of IκBα, thereby decreasing the level of NF-κB in the nucleus (Fig. 10). This enables one to predict that the *Rosa rugosa Thunberg* flower water extract inhibits the activation of STAT1 and NF-κB to decrease the expression level of the inflammatory cytokines, thereby leading to its anti-atopic effect.

Such results suggest that the *Rosa rugosa Thunberg* flower extract can treat IL-6-mediated diseases, and in particular, by effectively inhibiting the osteoclast differentiation, that it can prevent and treat bone metabolic diseases including osteoporosis, inflammatory bowel disease, and atopic dermatitis.

While the present invention has been described with reference to the particular illustrative embodiments, it will be understood by those skilled in the art to which the present invention pertains that the present invention may be embodied in other specific forms. In this regard, the exemplary embodiments disclosed herein are only for illustrative purposes and should not be construed as limiting the scope of the present invention.

## Claims

1. A pharmaceutical composition for use in preventing or treating an interleukin-6 (IL-6)-mediated disease, comprising a *Rosa rugosa Thunberg* flower water or ethanol extract as an active ingredient, wherein the IL-6-mediated disease is osteoporosis, inflammatory bowel disease, or atopic dermatitis.

2. The pharmaceutical composition for use of claim 1, wherein the extract inhibits an activity of signal transducer and activator of transcription 3 (STAT3).

3. The pharmaceutical composition for use of claim 1, wherein the extract inhibits osteoclast differentiation.

4. A food composition for use in preventing or improving an IL-6-mediated disease, comprising a *Rosa rugosa Thunberg* flower water or ethanol extract as an active ingredient, wherein the IL-6-mediated disease is osteoporosis, inflammatory bowel disease, or atopic dermatitis.

5. A feed composition for use in preventing or improving an IL-6-mediated disease, comprising a *Rosa rugosa Thunberg* flower water or ethanol extract as an active ingredient, wherein the IL-6-mediated disease is osteoporosis, inflammatory bowel disease, or atopic dermatitis.

6. A drinking water additive for use in preventing or improving an IL-6-mediated disease, comprising a *Rosa rugosa Thunberg* flower water or ethanol extract as an active ingredient, wherein the IL-6-mediated disease is osteoporosis, inflammatory bowel disease, or atopic dermatitis.

## Patentansprüche

1. Pharmazeutische Zusammensetzung zur Verwendung bei der Vorbeugung oder Behandlung einer durch Interleukin-6 (IL-6) vermittelten Erkrankung, umfassend einen Wasser- oder Ethanol-Extrakt der Blüte von *Rosa rugosa Thunberg* als aktiven Bestandteil, wobei die durch IL-6 vermittelte Erkrankung Osteoporose, eine entzündliche Darmerkrankung oder atopische Dermatitis ist.

2. Pharmazeutische Zusammensetzung zur Verwendung nach Anspruch 1, wobei der Extrakt eine Aktivität von STAT3 (signal transducer and activator of transcription 3) hemmt.

3. Pharmazeutische Zusammensetzung zur Verwendung nach Anspruch 1, wobei der Extrakt die Differenzierung von Osteoklasten hemmt.

4. Nahrungsmittel-Zusammensetzung zur Verwendung bei der Vorbeugung oder Besserung einer IL-6 vermittelten Erkrankung, umfassend einen Wasser- oder Ethanol-Extrakt der Blüte von *Rosa rugosa Thunberg* als aktiven Bestandteil, wobei die durch IL-6 vermittelte Erkrankung Osteoporose, eine entzündliche Darmerkrankung oder atopische Dermatitis ist.

5. Futtermittel-Zusammensetzung zur Verwendung bei der Vorbeugung oder Besserung einer durch IL-6 vermittelten Erkrankung, umfassend einen Wasser- oder Ethanol-Extrakt der Blüte von *Rosa rugosa Thunberg* als aktiven Bestandteil, wobei die durch IL-6 vermittelte Erkrankung Osteoporose, eine entzündliche Darmerkrankung oder atopische Dermatitis ist.

6. Trinkwasser-Additiv zur Verwendung bei der Vorbeugung oder Besserung einer durch IL-6 vermittelten Erkrankung, umfassend einen Wasser- oder Ethanol-Extrakt der Blüte von *Rosa rugosa Thunberg* als aktiven Bestandteil, wobei die durch IL-6 vermittelte Erkrankung Osteoporose, eine entzündliche Darmerkrankung oder atopische Dermatitis ist.

## Revendications

1. Composition pharmaceutique destinée à être utilisée dans la prévention ou le traitement d'une maladie médiée par l'interleukine-6 (IL-6), comprenant un extrait d'éthanol ou d'eau de fleur *Rosa rugosa de Thunberg* en tant que principe actif, dans laquelle la maladie médiée par l'IL-6 est l'ostéoporose, une maladie intestinale inflammatoire ou une dermatite atopique.

2. Composition pharmaceutique destinée à être utilisée selon la revendication 1, dans laquelle l'extrait inhibe une activité de transducteur de signal et d'activateur de transcription 3 (STAT3).

3. Composition pharmaceutique destinée à être utilisée selon la revendication 1, dans laquelle l'extrait inhibe la différenciation des ostéoclastes.

4. Composition d'aliment destinée à être utilisée dans la prévention ou l'amélioration d'une maladie médiée par l'IL-6, comprenant un extrait d'éthanol ou d'eau de fleur *Rosa rugosa de Thunberg* en tant que principe actif, dans laquelle la maladie médiée par l'IL-6 est l'ostéoporose, une maladie intestinale inflammatoire ou une dermatite atopique.

5. Composition d'aliment pour animaux destinée à être utilisée dans la prévention ou l'amélioration d'une maladie médiée par l'IL-6, comprenant un extrait d'éthanol ou d'eau de fleur *Rosa rugosa de Thunberg* en tant que principe actif, dans laquelle la maladie médiée par l'IL-6 est l'ostéoporose, une maladie intestinale inflammatoire ou une dermatite atopique.

6. Additif d'eau potable destiné à être utilisé dans la prévention ou l'amélioration d'une maladie médiée par l'IL-6, comprenant un extrait d'éthanol ou d'eau de fleur *Rosa rugosa de Thunberg* en tant que principe actif, dans laquelle la maladie médiée par l'IL-6 est l'ostéoporose, une maladie intestinale inflammatoire ou une dermatite atopique.
